# EUROPÄISCHE PATENTSCHRIFT

(11) **EP 0 535 480 B1**
(45) Veröffentlichungstag und Bekanntmachung des Hinweises auf die Patenterteilung: **15.05.1996**
(21) Anmeldenummer: 92116075.0
(22) Anmeldetag: 19.09.1992
(51) Int. Cl.: G01N 33/52, G01N 33/543

(54) **Testträger für die Analyse von Flüssigkeiten**
Slides for use in the analysis of liquids
Support de test pour l'analyse de liquides

(30) Priorität: 02.10.1991 DE 4132743
(43) Veröffentlichungstag der Anmeldung: 07.04.1993
(73) Patentinhaber: BOEHRINGER MANNHEIM GMBH, D-68298 Mannheim (DE)
(72) Erfinder: Krause, Manfred, Dipl.-Ing., W-6806 Viernheim (DE); Schäfer, Gerd Karl Peter, W-6700 Ludwigshafen (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 119 623
- EP-A- 0 452 740

## Beschreibung

Die Erfindung betrifft einen Testträger für die Analyse von Flüssigkeiten, der ein in einem Rahmen gehaltenes Testfeld mit einer Probenauftragsfläche zum Aufbringen einer Probenflüssigkeit aufweist, sowie ein Verfahren zur Herstellung eines solchen Testträgers.

Zur qualitativen oder quantitativen analytischen Bestimmung von Bestandteilen von Flüssigkeiten, insbesondere Körperflüssigkeiten von Menschen oder Tieren, werden zunehmend sogenannte Testträger verwendet. Sie enthalten die für die Analyse erforderlichen Reagenzien in einer oder mehreren Testschichten in trockener Form und werden in der englischen Literatur deswegen auch als "solid state analysis devices" bezeichnet. Die Probe wird auf eine Probenauftragsfläche aufgebracht und die Reaktion von Probe und Reagenzien führt zu einem nachweisbaren Signal, insbesondere einer Farbänderung, welche visuell oder mit Hilfe eines Gerätes, meistens reflexionsphotometrisch, ausgewertet werden kann.

Es sind zahlreiche verschiedene Testträgertypen bekannt, die sich nicht nur bezüglich der verwendeten Reagenzien, sondern auch durch ihren Aufbau, insbesondere hinsichtlich der Anordnung und Befestigung der Testschichten, unterscheiden. Insbesondere kann man folgende zwei grundsätzlich verschiedene Testträgertypen differenzieren.

Streifenförmige Testträger (Teststreifen) bestehen im wesentlichen aus einer länglichen Tragschicht aus Kunststoffmaterial und darauf befestigten Testschichten. Die Verbindung zwischen den Testschichten und dem Kunststoffträger wird meist durch Kleben hergestellt, wobei die Verklebung vielfach nicht auf der ganzen Fläche, sondern nur an einer Kante der Testschicht erfolgt. Gelegentlich werden auch zusätzlich Befestigungsmittel wie perforierte Folien oder Netze eingesetzt, die an der Tragschicht befestigt sind und die Testschichten indirekt festhalten. Die Testschichten können dabei unabhängig voneinander hergestellt und bei der Endmontage des streifenförmigen Testträgers entsprechend den Erfordernissen der jeweiligen Analyse angeordnet und befestigt werden.

Bei dem zweiten Testträgertyp wird ein Testfeld, welches die Reagenzien enthält, ähnlich wie ein photographisches Diapositiv von einem Rahmen gehalten. Solche Testträger werden im folgenden als "Testträger mit Rahmen" oder "Analysechips" bezeichnet. In der englischsprachigen Literatur ist der Begriff "analysis slides" gebräuchlich.

Das Testfeld der Testträger mit Rahmen besteht üblicherweise aus einem durchsichtigen Trägermaterial, auf dem ähnlich den lichtempfindlichen Schichten eines photographischen Films eine oder mehrere Reagenzschichten übereinander beschichtet sind, so daß sie vollflächig und fest miteinander verbunden sind. Das Testfeld ist somit ein einziges Teil, welches problemlos in einem Rahmen aus Kunststoff oder wasserfester Pappe eingerahmt werden kann.

Ein Vorteil der Analysechips ist darin zu sehen, daß der Rahmen den Transport und die Positionierung bei der automatischen Durchführung von Analysen mit Hilfe von Analysegeräten erleichtert. Andererseits ist jedoch die Herstellung mit den bekannten Verfahren verhältnismäßig aufwendig. Dies gilt besonders, wenn das Testfeld als Testschichtpaket ausgebildet ist, welches aus mindestens zwei lose aufeinander liegenden Schichten besteht.

Der Erfindung liegt die Aufgabe zugrunde, einen Testträger mit Rahmen zur Verfügung zu stellen, der sich auch für Anwendungsfälle eignet, bei denen das Testfeld als Testschichtpaket ausgebildet ist, welches aus mindestens zwei lose aufeinander liegenden Schichten besteht. Er soll sich zugleich durch eine sehr geringe Dicke, einfache Handhabung und kostengünstige Herstellung auszeichnen.

Die Aufgabe wird bei einem Testträger der eingangs bezeichneten Art dadurch gelöst, daß in einem Basiskörper aus Kunststoff eine muldenförmige Vertiefung zur Aufnahme des Testfeldes vorgesehen ist und die Vertiefung oberhalb des Testfeldes von einem Haltenetz aus einem Kunststoffmaterial überspannt ist.

Ein solcher Testträger ist extrem einfach aufgebaut. Der Basiskörper hat eine sehr einfache Form und läßt sich leicht und kostengünstig aus einem thermoplastischen Kunststoff im Spritzgußverfahren herstellen. Er kann aus einer flachen Scheibe ohne irgendwelche komplizierten Profile bestehen. Lediglich die muldenförmige Vertiefung zur Aufnahme des Testfeldes muß eingeprägt sein. Selbstverständlich ist es jedoch möglich, zusätzliche Ausnehmungen oder andere plastische Formgebungen vorzusehen, die beispielsweise zum Transport des Analysechips in einem Gerät oder bei der Herstellung zweckmäßig sind.

Als zweites Teil des Rahmens ist lediglich das Haltenetz erforderlich, welches nur wenig größer ist als das Testfeld selbst. Da es aus einem kommerziell erhältlichen Kunststoffgewebe aus vorzugsweise monofilen Fasern besteht, sind die Materialkosten hierfür extrem gering.

Im Rahmen der Erfindung wurde gefunden, daß das Netz ein Testschichtpaket aus mehreren im wesentlichen lose (jedenfalls ohne vollflächige Verbindung) aufeinander aufliegenden Testschichten nicht nur zuverlässig festhält, sondern die für manche Kunststoff-Netzmaterialien typische Elastizität des Haltenetzes günstige Resultate bezüglich des Flüssigkeitskontaktes der Testschichten untereinander zur Folge haben kann. Es läßt sich ein gleichmäßiger Flüssigkeitstransfer zwischen den Testschichten und damit eine gute Analysegenauigkeit erreichen, während bei einer starren Fixierung eines Testfeldpaketes die unvermeidlichen Dicketoleranzen üblicher Testfelder zu einer stark unterschiedlichen Druckbelastung und infolgedessen zu einem ungleichmäßigen Flüssigkeitsübertritt führen können.

Im Rahmen der Erfindung ist es möglich, Testschichten aus sehr unterschiedlichen Materialien je nach der Anforderung des jeweiligen Tests zu sehr unterschiedlichen Testschichtpaketen schichtpaketen zu kombinieren. Beispielsweise können Faserverbundstrukturen (Gewebe oder Vliese) feinporige Kunststoffschichten (Membranen), Filmschichten aus porösen oder quellfähigen Filmen und Testschichten auf Basis von Papier miteinander kombiniert werden, um für jede Schicht die im jeweiligen Anwendungsfall vorteilhaften Eigenschaften zu nutzen.

Bei Teststreifen ist die Verwendung eines Netzes zur Abdeckung der Testfelder bereits seit langem bekannt (deutsche Patentschrift 2 118 455). Bei der Konstruktion und Herstellung von Teststreifen herrschen jedoch völlig andere Bedingungen als bei Analysechips.

Teststreifen werden in einem kontinuierlichen Prozeß hergestellt, bei dem auf ein Kunststoffträgerband, dessen Breite der Länge der späteren Teststreifen entspricht, die einzelnen Testfelder ebenfalls in Form eines Bandes (dessen Breite der Länge des jeweiligen Testfeldes entspricht), kontinuierlich aufgeklebt werden. Erst am Ende des Herstellungsprozeßes wird das Bandmaterial in die einzelnen Flächenstreifen quer zu seiner Längsrichtung zerschnitten. In diesen Herstellungsprozeß läßt sich die Befestigung des Netzes leicht integrieren: Nach dem Aufkleben der Testfelder wird ein zusätzliches Band aus dem Netzmaterial zugeführt und zwischen den einzelnen Testschichten mit dem Trägermaterial verklebt. Diese Verfahrensweise wird auch in der deutschen Patentschrift 2 118 555 beschrieben, wobei für die Befestigung des Netzes auf dem Trägerband verschiedene für die Teststreifenherstellung gebräuchliche Verfahren erwähnt werden. In der Praxis hat sich das sogenannte "Heißsiegeln" durchgesetzt, bei dem das Trägermaterial mit einer Haftschicht aus einem Schmelzkleber-Kunststoff versehen wird und das Netzmaterial unter Erhitzen der Schmelzkleberschicht mittels einer beheizten Walze in diese eingedrückt wird.

Demgegenüber werden Testträger mit Rahmen einzeln hergestellt, wobei der Rahmen üblicherweise - wie bei photographischen Diapositiven - aus zwei in ihrer Flächenausdehnung etwa gleichgroßen Hälften besteht. Bei der Montage wird das Testfeld in die untere Hälfte des Rahmens eingelegt und mit der oberen Rahmenhälfte festgeklemmt oder festgeklebt.

Abweichend von dieser üblichen Bauweise von Analysechips wird der Rahmen bei der vorliegenden Erfindung im wesentlichen nur von einem Bauteil, nämlich dem Basiskörper, gebildet. Das Testfeld wird in der muldenförmigen Vertiefung von einem extrem kleinen und leichten Bauteil, dem Haltenetz, festgehalten.

Hiermit sind offensichtlich erhebliche Probleme bei der Entwicklung eines für die kostengünstige Massenproduktion der erfindungsgemäßen Analysechips geeigneten Verfahrens verbunden. Diese werden durch das erfindungsgemäße Verfahren gelöst, bei dem das Testfeld in die muldenförmige Vertiefung des Basisteils eingelegt, ein Netzmaterial für das Haltenetz als kontinuierliche Materialbahn zugeführt und in einem Arbeitsgang das Haltenetz von der Netzmaterialbahn abgetrennt, über der Vertiefung plaziert und in einem der muldenförmigen Vertiefung benachbarten Flächenstreifen mit dem Material des Basiskörpers thermisch verprägt wird.

Der Ausdruck "thermisches Verprägen" bezeichnet dabei einen Vorgang, bei welchem mindestens eines der Materialien so weit erwärmt wird, daß es erweicht und gleichzeitig die beiden Materialien gegeneinander gedrückt werden, so daß unter Einwirkung von Druck und erhöhter Temperatur die beiden Bauteile ohne Verwendung eines Klebematerials fest verbunden werden. Vorzugsweise erweicht das thermoplastische Material des Basiskörpers bei einer Temperatur, welche für die Formbeständigkeit des Materials des Haltenetzes unkritisch ist, d.h. weder zu einer wesentlichen Erweichung noch zu einer sonstigen störenden Beeinträchtigung des Netzmaterials führt. Beim thermischen Prägen unter Anwendung von Druck und Temperatur dringen dabei die Fäden des Haltenetzes in die erweichte Masse des Basiskörpermaterials ein. Nach dem Abkühlen und Erstarren des Basiskörpermaterials sind sie darin weitgehend eingebettet und werden zuverlässig festgehalten.

Eine solche Verfahrensweise ist für andere Zwecke bekannt. Auch in dem deutschen Patent 2 118 455 wird am Rande die Möglichkeit erwähnt, daß die Trägerschicht eines Teststreifen selbst als Haftschicht dienen kann, wenn sie beispielsweise aus Polyvinylchlorid besteht. In diesem Fall könne die Verbindung mit einem Netzwerk durch unmittelbares Verschweißen oder durch Druck nach Anquellung der Oberfläche mit einem geeigneten Lösungsmittel, wie zum Beispiel Methylenchlorid, erfolgen. Diese theoretischen Überlegungen haben jedoch keinen Eingang in die Praxis gefunden.

Die Befestigung des Haltenetzes erfolgt bei der Erfindung in einem die muldenförmige Vertiefung vorzugsweise vollständig umgebenden Flächenstreifen, der bevorzugt als gegenüber der Oberfläche des Basiskörpers zurückspringender Absatz ausgebildet ist. Bei den bekannten Teststreifen kann durch die Verwendung eines speziellen Schmelzklebermaterials mit deutlich niedrigeren Temperaturen gearbeitet werden als bei der Erfindung, bei der der Basiskörper insgesamt aus einem einheitlichen Material ausreichender Stabilität gefertigt ist. Zudem erfolgt das Siegeln bei den Teststreifen nur auf zwei Seiten des Testfeldes sehr schnell an einem kontinuierlich transportierten Band mit Hilfe rollender Walzen.

Die Erfindung wird im folgenden anhand eines in den Figuren schematisch dargestellten Ausführungsbeispiels näher erläutert; es zeigen:
- Fig. 1: Einen erfindungsgemäßen Testträger in perspektivischer Darstellung;
- Fig. 2: einen Schnitt entlang der Linie II-II in Fig. 1;
- Fig. 3: eine Aufsicht auf einen Basiskörper vor der Montage.
- Fig. 4: eine teilweise Schnittdarstellung ähnlich Fig. 2, jedoch nur von einem Basiskörper vor der Verprägung;
- Fig. 5: einen Schnitt entlang der Linie IV-IV in Fig. 2;
- Fig. 6: eine perspektivische Prinzipdarstellung des erfindungsgemäßen Herstellungsverfahrens.

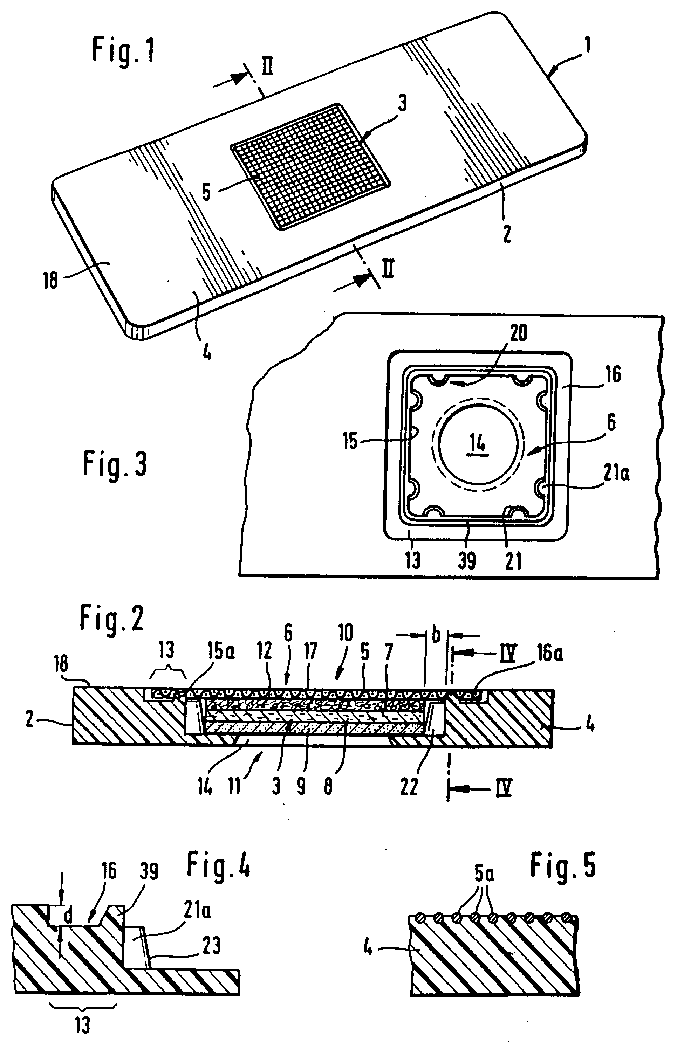

Der in Figur 1 dargestellte Testträger 1 hat einen flachen, vorzugsweise weniger als 3 mm, besonders bevorzugt maximal 2 mm dicken Rahmen 2, in dessen Mitte sich ein Testfeld 3 befindet. Der Rahmen 2 wird im wesentlichen von einem Basiskörper 4 gebildet. Das Testfeld 3 ist von einem Haltenetz 5 überspannt.

Einzelheiten der Konstruktion und Anordnung sind in den Figuren 2 und 3 dargestellt.

Der Basiskörper 4 weist eine muldenförmige Vertiefung 6 auf, in der das Testfeld 3 angeordnet ist. Das Testfeld 3 ist im dargestellten bevorzugten Fall als Testschichtpaket aus mehreren lose aufeinander liegenden Testschichten 7,8,9 ausgebildet. Die Schichten erfüllen verschiedene Funktionen im Testablauf. Beispielsweise kann die oberste Testschicht 7 eine Erythrozytenabtrennschicht sein, die zur Abtrennung der störenden roten Blutkörperchen aus der Blutprobe dient. Geeignet ist beispielsweise ein Glasfaservlies gemäß dem US-Patent 4 477 575. Die Schicht 8 kann beispielsweise eine mit einem Reagenz für eine Vorreaktion imprägnierte Papierschicht sein, während als unterste Testschicht beispielsweise ein Reagenzfilm verwendet werden kann, welcher ein Reagenzsystem enthält, das zu einer für die Analyse charakteristischen farblichen Veränderung auf der Unterseite der als Farbbildungsschicht bezeichneten Schicht 9 dient. Einzelheiten des Testablaufes sind für die vorliegende Erfindung ohne Bedeutung. Im Regelfall ist der Testablauf aber so, daß die Probe auf eine Seite des Testschichtpaketes, welche als Probenaufgabeseite 10 bezeichnet werden kann, auf eine Probenauftragsfläche 12 aufgegeben wird und die Messung bzw. visuelle Auswertung des Nachweissignals auf der gegenüberliegenden Seite des Testschichtpaketes (Nachweisseite 11) erfolgt.

Das Haltenetz ist in einem die muldenförmige Vertiefung 6 umgebenden Flächenstreifen 13 mit dem Basiskörper 4 thermisch verprägt. Bevorzugt ist es - wie dargestellt - auf der Probenaufgabeseite 10 des durch das Testschichtpaket 7,8,9 gebildeten Testfeldes 3 angeordnet. In diesem Fall kann es wichtige Zusatzfunktionen erfüllen. Es ist zweckmäßigerweise deutlich verschieden von dem Rahmen 2 gefärbt, um die Stelle, an der die Probe (beispielsweise ein Blutstropfen) aufgegeben werden muß, zu markieren.

Dies stellt einen Handhabungsvorteil insbesondere im Bereich der Selbstkontrolle durch Laien (beispielsweise Diabetiker) dar. Außerdem kann das Netz zu einer gleichmäßigen Verteilung der Probe auf die Probenauftragsfläche 12 des Testfeldes beitragen, wozu es zweckmäßigerweise mit einem Detergenz imprägniert sein kann. Für die Messung des Nachweissginals ist auf der Nachweisseite 11 in dem Basiskörper 4 eine Meßöffnung 14 vorgesehen.

In Ausnahmefällen kann im Rahmen der Erfindung jedoch auch eine umgekehrte Anordnung verwendet werden, bei der das Haltenetz 5 auf der Nachweisseite angeordnet ist und die Probenaufgabe auf der gegenüberliegenden Seite erfolgt. In jedem Fall wird als Oberseite des Analysechips in der vorliegenden Beschreibung diejenige Seite bezeichnet, auf der das Haltenetz 5 angebracht ist.

Der Basiskörper 4 besteht aus einer einfachen flachen Scheibe aus homogenem Kunststoff, beispielsweise Polystyrol, die im Spritzgießverfahren hergestellt ist. Die darin vorgesehene muldenförmige Vertiefung wird von einer Wand 15 begrenzt. An die Oberkante 15a der Wand 15 schließt sich nach außen hin ein umlaufender Absatz 16 an, der die muldenförmige Vertiefung 6 vollständig umgibt. Der Flächenstreifen 13, auf dem der Rand des Haltenetzes an dem Basiskörper befestigt ist, ist dabei ein Teil der horizontalen Fläche des Absatzes 16. Die Tiefe d des Absatzes 16 sollte etwa der Dicke des Haltenetzes 5 entsprechen oder etwas größer sein, damit die Oberfläche 17 des Haltenetzes 5 bündig mit der Oberfläche 18 des Basiskörpers 4 verläuft.

Die Oberfläche 18 des Basiskörpers 4 muß nicht notwendigerweise (wie dargestellt) frei von Unterbrechungen, wie zum Beispiel Vertiefungen oder Ausnehmungen sein. Wesentlich ist jedoch, daß im Bereich des Flächenstreifens 13 die Dicke des Basiskörpers 4 auf die Dicke des Testschichtpaketes 7,8,9 so abgestimmt ist, daß das gespannte Haltenetz 5 die Testschichten fixiert und erforderlichenfalls leicht zusammendrückt. Entsprechend ist der Begriff "muldenförmige Vertiefung zur Aufnahme des Testfeldes" in dem Sinne zu verstehen, daß es sich um eine flache Vertiefung innerhalb des Formteiles 4 handelt, deren Wände 15 etwa so hoch sind wie die Summe der genannten Schichten, so daß das gespannte Haltenetz leicht gegen die oberste Schicht drückt.

Im dargestellten bevorzugten Fall wird die horizontale Position des Testfeldes 3 innerhalb der muldenförmigen Vertiefung 6 nicht unmittelbar durch deren Wände 15 definiert. Vielmehr sind Positioniermittel 20 vorgesehen, die im dargestellten Fall durch diskrete Begrenzungselemente 21 in Form von von der Wand 15 ausgehenden halbsäulenförmigen Vorsprüngen 21a gebildet werden. Durch diese Positioniermittel wird das Testfeld 3 in der muldenförmigen Vertiefung 6 so positioniert, daß es auf dem größten Teil seines Umfangs allseitig von einem Spalt 22 umgeben ist, welcher vorzugsweise mindestens etwa 0,3 mm und höchstens etwa 1 mm breit ist.

Diese Maßnahme ist von besonderer Bedeutung, wenn das Testfeld aus mehreren Schichten besteht und verhindert werden muß, daß Probenflüssigkeit an den seitlichen Kanten des Testfeldpaketes 7,8,9 nach unten läuft, ohne die Testschichten in ihrer Reihenfolge von oben nach unten nacheinander zu passieren. Da für viele Reaktionen die korrekte Aufeinanderfolge der Kontaktierung der einzelnen Reagenzien bzw. die vorherige zuverlässige Abtrennung von störenden Bestandteilen erforderlich ist, würde durch einen derartigen "Probenkurzschluß" das Meßergebnis verfälscht. Überraschenderweise hat sich gezeigt, daß dies im Rahmen der vorliegenden Erfindung zuverlässig verhindert werden kann, obwohl die Fläche des Haltenetzes 5 größer ist als die Oberfläche des Testfeldes 3 und nicht ausgeschlossen werden kann, daß die Probe auch auf solche Bereiche des Haltenetzes 5 aufgebracht wird, die am Rande oder außerhalb des Testfeldes 3 liegen. Vorzugsweise ist die Fläche des Haltenetzes nur geringfügig größer als die Fläche des Testfeldes, wobei die Fläche des Haltenetzes bevorzugt zwischen 150 % und 500 %, besonders bevorzugt zwischen 180 % und 300 % der Fläche des Testfeldes 3 beträgt.

Statt der halbsäulenförmigen Vorsprünge 21a können andere diskrete Begrenzungselemente 21 als Positioniermittel eingesetzt werden, die beispielsweise einen dreieckigen Querschnitt aufweisen. Die Kontaktfläche der Begrenzungselemente, mit denen sie mit der äußeren Kante der Testschichten 7,8,9 in Kontakt stehen, sollte möglichst klein sein. Bevorzugt ist eine Form, die einen Linienkontakt gewährleistet. In jedem Fall sollte der umlaufende Spalt 22 höchstens auf 25 %, besonders bevorzugt auf weniger als 10 % seiner Länge von den Begrenzungselementen 21 unterbrochen werden.

Gemäß einer weiteren bevorzugten Ausführungsform sind die Begrenzungselemente 21 so ausgebildet, daß ihre dem Testfeld 3 zugewandte Seite 23 von unten nach oben schräg von dem Testfeld weg verläuft. Die Abstände der Begrenzungselemente 21 und die Abmessungen des Testfeldes 3 sind dabei so aufeinander abgestimmt, daß die untere Kante der untersten Schicht 9 des Testfeldes 3 an dem Begrenzungselement 21 anliegt. Dadurch wird eine sehr genaue Positionierung erreicht und zugleich eine einfache und rationelle maschinelle Montage ermöglicht. Vor allem die für die optische Auswertung wesentliche Farbbildungsschicht 9 wird sehr genau positioniert, während die darüberliegende Testschichten 7,8 mit einem gewissen Spiel positioniert werden können.

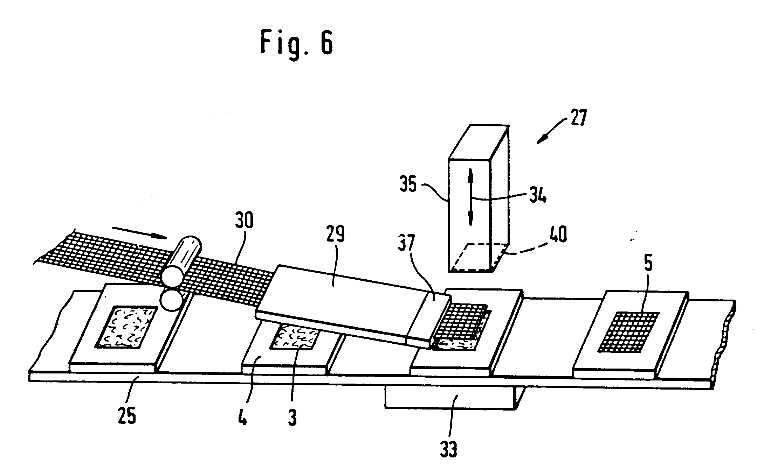

Die Figuren 3 bis 6 dienen der Erläuterung des erfindungsgemäßen Verfahrens bzw. der damit im Zusammenhang stehenden Konstruktionsmerkmale des Testträgers.

Basiskörper 4 werden taktweise über eine konventionell ausgebildete und im einzelnen nicht dargestellte Transportstrecke 25 transportiert. An einer nicht dargestellten Füllstation wird das Testfeld 3 in die muldenförmige Vertiefung eingelegt.

Danach wird der Analysechip zu der insgesamt mit 27 bezeichneten Verschließstation transportiert. Sie umfaßt einen Zuführkanal 29 durch den eine Bahn 30 von einer Rolle zugeführt wird, einen in die Transportstrecke 25 eingelassenen Amboß 33 und einen in Richtung des Pfeiles 34 vertikal auf- und abwärts beweglichen Prägestempel 35.

Der Prägestempel 35 dient zugleich dazu, ein passendes Stück von der Netzmaterialbahn 30 als Haltenetz 5 abzutrennen. Im dargestellten Fall hat die Netzmaterialbahn 30 die passende Breite, so daß nur noch jeweils ein in der Länge passendes Stück abgeschnitten werden muß. Dies geschieht bevorzugt durch Abstanzen, wobei die in Transportrichtung der Basiskörper 4 vordere Kante des Prägestempels 35 entsprechend scharfkantig ist und mit einem Untermesser 37 zusammenwirkt, welches das in Transportrichtung hintere Ende des Zuführkanals 29 bildet. Statt dessen könnte auch eine Materialbahn 30 von größerer Breite verwendet werden, wobei in diesem Fall das Untermesser 37 eine scharfkantige Ausnehmung in der Größe des gewünschten Haltenetzes 5 aufweist und der Prägestempel 35 allseitig scharfkantig ausgebildet sein muß, um ein passendes Stück aus der Netzmaterialbahn 30 auszustanzen.

Wesentlich ist, daß in einem Arbeitsgang unmittelbar nach dem Abtrennen des Haltenetzes 5 die thermische Verprägung in dem die muldenförmige Vertiefung umgebenden Flächenstreifen 13 des Basiskörpers stattfindet. Zu diesem Zweck ist ein gestrichelt angedeuteter umlaufender Prägewulst 40 entsprechender Breite am Rande der unteren Begrenzungsfläche des Prägestempel 35 beheizt. Er drückt den Rand des Haltenetzes 5 gegen den Flächenstreifen 13, in dem die Verprägung stattfinden soll. Der Rand des Haltenetzes 5 wird in das erweichende Material des Basiskörpers 4 eingedrückt, so daß die einzelnen Fäden 5a größtenteils in das Material eingebettet werden, wie dies in Figur 5 dargestellt ist. Um dies zu erleichtern, sollte das Netzmaterial nicht zu dicht sein. Vorzugsweise sollte der Abstand der Fäden mindestens halb so groß, besonders bevorzugt mindestens ebenso groß wie der Durchmesser der Fäden sein.

Um die Verprägung weiter zu erleichtern und die thermische Belastung der Testfelder zu reduzieren ist es vorteilhaft, wenn innerhalb des Prägestreifens 13 vor der Verprägung ein umlaufender nach oben vorspringender Wulst 39 vorgesehen ist (Fig. 4). Er sollte vorzugsweise mindestens etwa 0,2 mm, besonders bevorzugt etwa 0,3 bis 0,4 mm hoch sein.

## Patentansprüche

1. Testträger für die Analyse von Flüssigkeiten, der ein in einem Rahmen (2) gehaltenes Testfeld (3) mit einer Probenauftragsfläche (12) zum Auftragen einer Probenflüssigkeit aufweist, **dadurch gekennzeichnet**, daß in einem Basiskörper (4) aus Kunststoff eine muldenförmige Vertiefung (6) zur Aufnahme des Testfeldes (3) vorgesehen ist und die Vertiefung (6) oberhalb des Testfeldes (3) von einem Haltenetz (5) aus einem Kunststoffmaterial überspannt ist.

2. Testträger nach Anspruch 1, **dadurch gekennzeichnet**, daß das Testfeld (3) als Testschichtpaket mit mindestens zwei aufeinander liegenden, nicht vollflächig miteinander verbundenen Testschichten (7,8,9) ausgebildet ist.

3. Testträger nach einem der vorhergehenden Ansprüche, **dadurch gekennzeichnet**, daß die von dem Haltenetz (5) überspannte Oberfläche des Testfeldes die Probenauftragsfläche (12) ist.

4. Testträger nach einem der vorhergehenden Ansprüche, **dadurch gekennzeichnet**, daß das Material des Basiskörpers (4) thermoplastisch ist und bei einer für die Formbeständigkeit des Haltenetzes (5) unkritischen Temperatur erweicht und daß das Netz an seinem Rand durch lokales Erweichen eines der muldenförmigen Vertiefung (6) benachbarten Flächenstreifens (13) des Basiskörpers (4) und durch Eindrücken in das erweichte Material befestigt ist.

5. Testträger nach Anspruch 4, **dadurch gekennzeichnet**, daß der Flächenstreifen (13) die muldenförmige Vertiefung (6) vollständig umgibt.

6. Testträger nach einem der vorhergehenden Ansprüche, **dadurch gekennzeichnet**, daß die die muldenförmige Vertiefung (6) begrenzende Wand (15) an ihrer Oberkante (15a) in einen gegenüber der Oberfläche (18) des Basiskörpers (4) zurückspringenden Absatz (16) übergeht.

7. Testträger nach einem der vorhergehenden Ansprüche, **dadurch gekennzeichnet**, daß die muldenförmige Vertiefung (6) größer als das Testfeld (3) ist und Positioniermittel (20) vorgesehen sind, die das Testfeld (3) in der muldenförmigen Vertiefung (6) so positionieren, daß es auf dem größten Teil seines Umfanges allseitig von einem Spalt (22) umgeben ist.

8. Testträger nach Anspruch 7, **dadurch gekennzeichnet**, daß der Spalt (22) mindestens 0,3 mm und höchstens 1 mm breit ist.

9. Verfahren zum Herstellen eines Testträgers nach Anspruch 1, **dadurch gekennzeichnet**, daß das Testfeld in die muldenförmige Vertiefung des Basiskörpers eingelegt, ein Netzmaterial für das Haltenetz als kontinuierliche Materialbahn zugeführt und in einem Arbeitsgang das Haltenetz von der Netzmaterialbahn abgetrennt, über der muldenförmigen Vertiefung plaziert und in einem die muldenförmige Vertiefung umgebenden Flächenstreifen mit dem Material des Basiskörpers thermisch verprägt wird.

10. Verfahren nach Anspruch 9, **dadurch gekennzeichnet**, daß in dem Flächenstreifen des Basiskörpers, in dem die thermische Verprägung stattfindet, vor der Verprägung ein umlaufender, nach oben vorspringender Wulst (39) vorhanden ist.

## Claims

1. Test carrier for the analysis of fluids, comprising a test field (3) which is held in a frame (2) and has a sample application surface (12) for the application of a sample fluid, **characterised in that** in a base body (4) of plastics material a trough-shaped depression (6) for accommodating the test field (3) is provided and the depression (6) is spanned above the test field (3) by a retaining net (5) of a plastics material.

2. Test carrier according to claim 1, **characterised in that** the test field (3) is provided as a test layer pack with at least two test layers (7, 8, 9) lying on one another and not connected to one another over their whole surface.

3. Test carrier according to any one of the preceding claims, **characterised in that** the surface of the test field which is spanned by the retaining net (5) is the sample application surface (12).

4. Test carrier according to any one of the preceding claims, **characterised in that** the material of the base body (4) is thermoplastic and softens at a temperature not critical for the dimensional stability of the retaining net (5) and that the net is fixed at its edge by local softening of a surface strip (13) of the base body (4) which is adjacent to the trough-shaped depression (6) and by pressing the net into the softened material.

5. Test carrier according to claim 4, **characterised in that** the surface strip (13) completely surrounds the trough-shaped depression (6).

6. Test carrier according to any one of the preceding claims, **characterised in that** the wall (15) limiting the trough-shaped depression (6) passes over at its top edge (15a) into a shoulder (16) recessed with respect to the surface (18) of the base body (4).

7. Test carrier according to any one of the preceding claims, **characterised in that** the trough-shaped depression (6) is greater than the test field (3) and positioning means (20) are provided which position the test field (3) in the trough-shaped depression (6) so that it is surrounded on all sides on the major part of its periphery by a gap (22).

8. Test carrier according to claim 7, **characterised in that** the gap (22) is at least 0.3 mm and at most 1 mm wide.

9. Method for manufacturing a test carrier according to claim 1, **characterised in that** the test field is introduced into the trough-shaped depression of the base body, a net material for the retaining net is fed as a continuous band of material and in one working step the retaining net is separated from the band of net material, placed above the trough-shaped depression and thermally bonded with the material of the base body in a surface strip surrounding the trough-shaped depression.

10. Method according to claim 9, **characterised in that** in the surface strip of the base body in which the thermal bonding takes place there is provided prior to the bonding a peripheral, upwardly projecting bead (39).

## Revendications

1. Support de test pour l'analyse de liquides, qui est constitué d'une zone de test (3) maintenue dans un cadre (2) avec une surface de dépôt d'échantillon (12) pour porter un échantillon liquide, caractérisé en ce qu'on prévoit dans un corps de base (4) de matière plastique une dépression (6) en forme d'auge pour le logement de la zone de test (3) et que la dépression (6) est recouverte au-dessus de la zone de test (3) par un maillage de maintien (5) de matière plastique.

2. Support de test selon la revendication 1, caractérisé en ce que la zone de test (3) est constituée sous la forme d'une superposition de couches de test avec au moins deux couches de test superposées, ne se joignant pas sur la surface totale.

3. Support de test selon l'une quelconque des revendications précédentes, caractérisé en ce que la surface recouverte par le maillage de maintien (5) de la zone de test est la surface de dépôt d'échantillon (12).

4. Support de test selon l'une quelconque des revendications précédentes, caractérisé en ce que la matière du corps de base (4) est thermoplastique et se ramollit à une température non critique pour la stabilité de la forme du maillage de maintien (5) et que le maillage est fixé en son pourtour par un ramollissement d'une bande de surface (13) adjacente à la dépression (6) en forme d'auge du corps de base (4) et par enfoncement dans la matière ramollie.

5. Support de test selon la revendication 4, caractérisé en ce que la bande de surface (13) entoure complètement la dépression en forme d'auge (6).

6. Support de test selon l'une quelconque des revendications précédentes, caractérisé en ce qu'une paroi (15) limitant la dépression en forme d'auge (6) en son bord supérieur est entourée par une gorge (16) en creux par rapport à la surface (18) du corps de base (4).

7. Support de test selon l'une quelconque des revendications précédentes, caractérisé en ce que la dépression en forme d'auge (6) est plus grande que la zone de test (3) et que des moyens de positionnement sont prévus pour positionner la zone de test (3) dans la dépression en forme d'auge (6) de telle sorte qu'elle est entourée d'un espacement (22) de tous les côtés sur la plus grande partie de son périmètre.

8. Support de test selon la revendication 7, caractérisé en ce que l'espacement (22) a une largeur d'au moins 0,3 mm et au plus de 1 mm.

9. Procédé pour la fabrication d'un support de test selon la revendication 1, caractérisé en ce que la zone de test est introduite dans la dépression en forme d'auge du corps de base, une matière maillée est introduite pour le maillage de maintien sous forme d'une nappe de tissu continue et en une seule opération le maillage de maintien est séparé de la nappe de maillage, est placé au-dessus de la dépression en forme d'auge, est pressé à chaud contre la matière du corps de base pour former une bande entourant la dépression en forme d'auge.

10. Procédé selon la revendication 9, caractérisé en ce que dans la bande de surface du corps de base, dans laquelle a lieu le pressage à chaud, avant le pressage se trouve un rebord (39) se projetant vers le haut.
